# EUROPÄISCHE PATENTANMELDUNG

(11) **EP 1 129 678 A2**
(43) Veröffentlichungstag der Anmeldung: **05.09.2001**
(21) Anmeldenummer: 01104245.4
(22) Anmeldetag: 22.02.2001
(51) Int. Cl.: A61F 2/78

(54) **Prothesenfestes Verriegelungselement für eine Verriegelung zwischen einem über einen Amputationsstumpf zu ziehenden Strumpf und einer Prothese**

(30) Priorität: 02.03.2000 DE 10009968
(71) Anmelder: Heggemann GmbH, 33142 Büren (DE)
(72) Erfinder: Lührs, Bernd, 33142 Büren (DE); Theile, Frank, 34519 Diemelsee Adorf (DE); Griesser, Michael, 33154 Salzkotten (DE)
(74) Vertreter: Jäger, Jörg

(57) **Zusammenfassung**

Um den Tragekomfort insbesondere einer Unter- oder Oberschenkelprothese zu erhöhen, wird ein prothesenfestes Verriegelungselement für eine Verriegelung zwischen einem über einen Amputationsstumpf zu ziehenden Strumpf und einer Prothese, mit einer Einzugsmechanik 1 für einen an dem Strumpf fest angeschlossenen Verriegelungsstift mit einer eine Relativbewegung zwischen der Einzugsmechanik 1 und einer prothesenfesten Aufnahme 2 zulassenden Verbindung versehen.

## Beschreibung

Die Erfindung betrifft ein prothesenfestes Verriegelungselement für eine Verriegelung zwischen einem über einen Amputationsstumpf zu ziehenden Strumpf und einer Prothese, mit einer Einzugsmechanik für einen an dem Strumpf fest angeschlossenen Verriegelungsstift.

Bei bekannten Verriegelungssystemen rollt der Prothesenträger sich einen aus einem Silikon hergestellten, sackartigen Strumpf, einen sogenannten Silikon-Liner, über den Stumpf. Mittels dieses Strumpfes werden Kräfte von einer Prothese auf den Stumpf über einen in dem Strumpfboden befestigten Verriegelungsstift und ein prothesenfestes Verriegelungselement übertragen, wenn der Verriegelungsstift in eine Aufnahme einer Einzugsmechanik eingeführt, eingezogen und verriegelt ist. Dieses Verriegelungssystem wird regelmäßig als Clutch-Lock auch bezeichnet. Das Einziehen des eine Zahnung aufweisenden Verriegelungsstiftes in die Aufnahme erfolgt mittels einer eine entsprechende Zahnung aufweisenden Schneckenkupplung einer Einzugsvorrichtung, von der der Verriegelungsstift, zumeist als Verriegelungs-Pin bezeichnet, von außen mit einer Drehbewegung in die Aufnahme des Verriegelungssystems für einen sicheren Sitz eingezogen werden kann und in dem er dann verriegelt wird. Diese Einzugsmechanik mit Einzugs- und Ver- bez.

Entriegelungsvorrichtung für den Verriegelungsstift in der Aufnahme ist nach dem Stand der Technik unbeweglich mit der Prothese verbunden, so daß eine feste Anbindung der Prothese über dem Silikon-Liner mit dem Stumpf gegeben ist. Diese starre Verbindung, lediglich etwas durch die Flexibilität des Strumpfes gemindert, verhindert letztlich, daß auftretende Zugkräfte die Prothese von dem Stumpf des Prothesenträgers lösen können. Über einen Entriegelungsknopf der Entriegelungsvorrichtung kann die Verriegelung zwischen der Verriegelungsvorrichtung der Einzugsmechanik und dem Verriegelungsstift wieder gelöst werden und ist danach der Patient in der Lage, den Strumpf bzw. den Silikon-Liner von der Prothese abzunehmen, womit Strumpf und Prothese wieder getrennt sind.

Durch die feste Verbindung zwischen der Prothese und dem Strumpf werden alle Kräfte, bspw. hervorgerufen durch das Abkippen des Verriegelungsstiftes beim Anbeugen des Stumpfes, über den Silikon-Liner auch auf den Stumpf übertragen. Diese Zug- und Schiebekräfte sind zum einen für einen Prothesenträger äußerst unangenehm und können leicht, z. B. bei einem Rutschen des Silikon-Liners, zu Druckstellen, wunden Stellen oder dergleichen führen.

Vor dieser technischen Problematik der Anbindung einer Prothese an einen Stumpf stellt sich die Aufgabe, ein Verriegelungselement zur Verfügung zu stellen, das insbesondere geringfügige, wie beim Abkippen des Verriegelungsstiftes beim Anbeugen des Stumpfes, hervorgerufene, Zug- und Schiebekräfte minimiert.

Gelöst wird diese technische Problematik durch den Gegenstand des Anspruches 1, bei dem bei einem gattungsgemäßen Verriegelungselement auf eine eine relative Bewegung zwischen der Einzugsmechanik und einer prothesenfesten Aufnahme zulassenden Verbindung abgestellt ist.

Die Einzugsmechanik für den Verriegelungsstift bzw. einen Verriegelungs-Pin kann dem Stand der Technik nach wie vor entsprechen. Wesentlich ist die Möglichkeit einer Relativbewegung zwischen dieser Einzugsmechanik und der prothesenfesten Aufnahme. Hierdurch ist die Möglichkeit einer Beweglichkeit der Prothese gegenüber dem Stumpf zugelassen, die nicht über den in der Aufnahme der Einzugsmechanik festgehaltenen Verriegelungsstift bzw. Verriegelungs-Pin auf den Silikon-Liner und damit auf den Stumpf mit den entsprechend auftretenden Kräften übertragen wird. Vielmehr wird bewußt zugelassen, daß eine Relativbewegung auch stattfinden darf. Diese sollte naturgemäß nicht beliebig sein, sondern letztlich durch geeignete Maßnahmen begrenzt werden, so daß die normale Funktion der Prothese gewahrt bleibt.

Insbesondere hat es sich als zweckmäßig erwiesen, wenn die Relativbewegung eine Bewegung mit drei Freiheitsgraden ist. Hierdurch wird sichergestellt, daß in alle Richtungen eine Relativbewegung auch erfolgen kann und eine solche Relativbewegung bei jeder Bewegung des Stumpfes auch stattfinden kann.

Als äußerst zweckmäßig hat sich hierbei eine Relativbewegung erwiesen, die wenigstens eine rotationssymmetrische, insbesondere auch eine kreissymmetrische Bewegungskomponente aufweist. Durch solche Bewegungen kommt es zu keiner räumlichen Verlagerung, sondern führen diese im wesentlichen lediglich zu einer Art Abknicken der Prothese an der Verbindung und damit gegenüber dem Stumpf.

Bereits die Maßnahme der zugelassenen -insbesondere beschränkten- Relativbewegung verschaffen dem Prothesenträger erhebliche Erleichterungen. Der Tragekomfort wird jedoch weiter erhöht, wenn diese mögliche Relativbewegung, insbesondere sofort mit Bewegungsbeginn, abgebremst wird. Es kommt durch diese Maßnahme die Prothese in ihrer Bewegung gleichsam von selbst in eine Ruhelage, wobei äußerst geringe Kräfte nur auf den Strumpf übertragen werden. Diese Kräfte sind von daher auch weitaus weniger den Stumpf belastend als bei einem Clutch-Lock nach dem Stand der Technik.

Als äußerst zweckmäßig hat es sich erwiesen, wenn die Relativbewegung durch Reibungskraft abgebremst wird. Diese Maßnahme hat zur Folge, daß bei der Verbindung Verzögerungselemente, Bremselemente oder dergleichen mehr nicht gesondert ausgebildet werden müssen und demzufolge das Volumen des prothesenfesten Verriegelungselementes vergleichsweise klein auch gehalten werden kann.

Die bereits angesprochene Begrenzung der Relativbewegung sollte insbesondere bei einer rotationssymmetrischen bzw. bei einer Kreisbewegung bei einem Gesamtöffnungswinkel von 30° insgesamt etwa liegen, wodurch gegenüber einer Symmetrieachse bei einer symmetrischen Anordnung jeweils eine Auslenkung von etwa 15° ermöglicht ist.

In technischer Ausgestaltung des prothesenfesten Verriegelungselementes ist vorgesehen, daß die Verbindung ein Gelenk ist, insbesondere ein kardanisches Gelenk, das Relativbewegungen mit drei Freiheitsgraden auch erlaubt. Hierbei ist insbesondere daran gedacht, daß die Verbindung als Kugelgelenk ausgeführt ist, wodurch rotationssymmetrische bzw. Kreisbewegungen in allen Freiheitsgraden als Relativbewegung ermöglicht werden. Aus Platzgründen und für eine einfache Begrenzung des Ausschlages eines solchen Gelenkes, z. B. auf 30°, kann in weiterer Ausgestaltung eine Verbindung als Kugelschichtgelenk vorgesehen sein. Ein solches Kugelschichtgelenk entspricht weitgehend in der Funktion einem Kugelgelenk, jedoch fehlen dem von Kugelschalenabschnitten aufgenommenen, kugelschichtartig ausgebildeten Gelenkkörper gegenüberliegende Polkappen auf einer Symmetrieachse, die auch Drehachse ist.

Bei einer bevorzugten Ausführungsform ist vorgesehen, daß die Aufnahme ein Ober- und ein Unterteil mit jeweils halbschalenartig ausgebildeten Gleitflächen aufweist zur Aufnahme eines rotationssymmetrischen, insbesondere kugelartigen, die Einzugsmechanik aufweisenden Gelenkkörpers. Hierdurch ist ein Gelenk verwirklicht, das vergleichsweise klein ausgebildet ist und bei dem die Möglichkeit besteht, herkömmliche Einzugsmechaniken auch weiter zu verwenden. Es ist sogar möglich, bisherige Silikon-Liner weiter zu verwenden und lediglich die Prothese mit dem Verriegelungselement nach der Erfindung zu versehen. Die Vorteile des erfindungsgemäßen Verriegelungselementes können auch durch ein Umrüsten einer vorhandenen Prothese zum Tragen kommen.

In weiterer technischer Ausgestaltung des Verriegelungselementes nach der Erfindung ist vorgesehen, daß Ober- und Unterteil miteinander verschraubt sind in der Art, daß über die Schrauben die Reibungskraft zwischen den Gleitflächen und dem Gelenkkörper einstellbar ist. Durch diese Maßnahme wird eine Bewegung der Prothese gegenüber dem Stumpf zunächst sofort, aber regelmäßig nicht vollständig, abgebremst. Entsprechend gering sind die dann übertragenen Kräfte. Diese Justiermöglichkeit der die Relativbewegung abdämpfenden Kraft erlaubt weiter eine individuelle Anpassung und Abstimmung auf einen Prothesenträger bzw. die Prothese. Eine besonders feine Einstellmöglichkeit ist dann gegeben, wenn in weiterer konstruktiver Ausgestaltung vorgesehen ist, daß die Schrauben jeweils gegen die Kraft einer Feder einschraubbar sind. Neben dem feinfühligen Feststellen dieser Schrauben sind diese weitgehend gegen ein Lockern dann auch gesichert. Für die Verbesserung der Einstellmöglichkeiten der aufzubringenden Reibungskraft kann weiter zwischen dem Ober- und dem Unterteil wenigstens eine die Gleitflächen trennende, umlaufenden Nutung vorgesehen sein. Eine derartige Ausnehmung kann entweder das Ober- oder das Unterteil aufweisen oder können beide entsprechende Nutungen tragen. Ziel der Maßnahme ist es, den Bereich der Gleitflächen zwischen Ober- und Unterteil durch einen Schlitz beabstandet zu trennen.

In weiterer technischer Ausgestaltung des Verriegelungselementes nach der Erfindung ist vorgesehen, daß eine Symmetrie- und Drehachse des Gelenkkörpers und die Achse einer stiftartig ausgebildeten Einzugs- und Ver- bez. Entriegelungsvorrichtung der Einzugsmechanik Normale senkrecht aufeinander stehender Ebenen sind. Hierdurch ist zum einen eine gute Krafteinleitung von dem Verriegelungs-Pin über die Einzugsmechanik auf den Gelenkkörper gegeben und zum anderen für die Relativbewegung eine exakte Orientierung der Bewegungsrichtungen.

In weiterer konstruktiver Ausgestaltung ist vorgesehen, daß das Ober- und/oder das Unterteil eine Durchbrechung ihrer Mantelwand aufweisen für die Durchführung der Einzugsvorrichtung in Form bspw. eines Schneckenrades und/oder einer Ver- und Entriegelungsvorrichtung, im Stand der Technik regelmäßig stiftartig ausgebildet, für den Verriegelungsstift. Hierbei wird zweckmäßigerweise durch das Umfangsmaß der Durchbrechung eine Rotation des Gelenkkörpers um seine Symmetrie- und Drehachse begrenzt. Dies erfolgt durch einen Anschlag einer Führung für und/oder einer stiftartig ausgebildeten Einzugsvorrichtung bzw. Ver- und Entriegelungsvorrichtung selbst gegen insbesondere senkrecht aufgehende Ränder der Begrenzung der Durchbrechung(en).

Weiter ist vorgesehen, daß das Oberteil eine Durchbrechung aufweist, die von einem Ansatz des Gelenkkörpers durchsetzt wird. Auch durch diesen Ansatz, die Durchbrechung des Oberteiles durchsetzend, wird die Relativbewegung begrenzt. Insbesondere wird durch diese Maßnahme eine Kippbewegung durch den Anschlag des Ansatzes an den Rand der Durchbrechung begrenzt. Als zweckmäßig hat es sich hierbei weiter erwiesen, wenn der Ansatz und die Durchbrechung des Oberteiles rotationssymmetrisch, insbesondere kreissymmetrisch ausgebildet sind, insbesondere symmetrisch zu der bereits erwähnten Symmetrie- und Drehachse des Gelenkkörpers. Durch diese Maßnahmen wird auch die Relativbewegung symmetrisch begrenzt.

Weiter kann der Ansatz selbst die Aufnahme ausbilden oder an eine solche angeschlossen sein, die der Aufnahme des Verriegelungsstiftes dient und/oder eine Auflage für den Stumpf ausbilden.

Vorteilhafterweise ist die Aufnahme des Gelenkkörpers und/oder der Gelenkkörper selbst aus einem Kunststoff, insbesondere aus einem Polyamid, bestehend ausgeführt. Hierdurch wird ein geringes Gewicht des Verriegelungselementes erreicht. Gleichzeitig ist es jedoch äußerst stabil, ohne spröde zu sein.

Das Verriegelungselement nach der Erfindung kann eine äußerst geringe Bauhöhe dann aufweisen, wenn auf eine Schneckenkupplung mit einem Schneckenrad von vergleichsweise großem Durchmesser der Einzugsvorrichtung verzichtet wird. Bevorzugt wird dann eine Einzugsmechanik, die eine einen in eine Aufnahme eingebrachten Verriegelungsstift gegen ein Ausziehen klemmend haltende Verriegelungsvorrichtung aufweist. Ein solcher insbesondere dann auch glatt ausgebildeter Verriegelungsstift kann bspw. nach Art der Verriegelung bei den bekannten Pistolen für Kartuschen für Kleb- oder Schaumstoffe ausgebildet sein, bei denen der den Boden der Kartusche einschiebende, von einem Abzug in Vorschub gebrachte, häufig glatte Stab in jeder vorgeschobenen Position sofort arretiert wird und nicht mehr zurückgezogen werden kann.

Für ein sicheres Arretieren durch ein Klemmen kann vorgesehen sein, daß die Verriegelungsvorrichtung einen Klemmbacken, einen Klemmbügel oder dergleichen aufweist, der durch eine Keilwirkung den Verriegelungsstift in seiner Aufnahme selbsttätig klemmend festlegt. Durch eine Keilwirkung können sehr große klemmende und damit den Verriegelungsstift festlegende Kräfte hervorgerufen werden, die sicher ein unbeabsichtigtes Ausziehen des Verriegelungsstiftes ausschließen.

Um das Verriegelungselement wieder von dem Verriegelungsstift lösen zu können, ist regelmäßig eine den Klemmbacken, den Klemmbügel oder dergleichen lösende Entriegelungsvorrichtung vorgesehen, die aus einem einfachen Zug- oder Druckelement bestehen kann, da einfacher Zug oder Druck den Klemmbacken, den Klemmbügel oder dergleichen aus seiner Keillage bringen kann, womit dessen Klemmwirkung aufgehoben wird.

Mit Fortfall des Schneckengetriebes ist auch der Einzug des Verriegelungsstiftes in seine Aufnahme mit dessen Hilfe nicht mehr möglich. Alternativ, sofern gewünscht und das Einbringen des Verriegelungsstiftes unter Gewichtskraft nicht ausreichend sein sollte, kann durch eine an dem Verriegelungsstift angebundene Zugvorrichtung, die durch einen prothesenfesten Auslaß geführt wird, ein weitgehendes Einziehen des Verriegelungsstiftes in seine Aufnahme sichergestellt werden, da durch Zug an dem freien Ende der Zugvorrichtung, einem Seil, einem Draht oder dergleichen, dieses Einziehen sichergestellt werden kann.

Die Erfindung wird anhand der Zeichnung näher erläutert, in der lediglich ein Ausführungsbeispiel dargestellt ist. In der Zeichnung zeigt:
- Fig. 1:: in einer isometrischen Darstellung ein Verriegelungselement nach der Erfindung,
- Fig. 2:: das Verriegelungselement nach Fig. 1 in einer geschnittenen Darstellung,
- Fig. 3:: eine Ansicht gemäß Fig. 2 mit einer maximalen Auslenkung des Gelenkkörpers,
- Fig. 4:: eine Draufsicht auf ein Oberteil einer Aufnahme,
- Fig. 5:: einen Schnitt gemäß der Linie V,V in Fig. 4,
- Fig. 6:: einen Schnitt durch ein Unterteil einer Aufnahme gemäß der Linie VI,VI in Fig. 7,
- Fig. 7:: eine Draufsicht auf ein Unterteil,
- Fig. 8:: eine seitliche Ansicht eines Gelenkkörpers,
- Fig. 9:: eine Draufsicht auf den Gelenkkörper nach Fig. 8,
- Fig.10:: einen Schnitt gemäß der Linie X,X in Fig. 8 durch einen Gelenkkörper und
- Fig.11:: einen Schnitt gemäß der Linie XI, XI in Fig. 9.

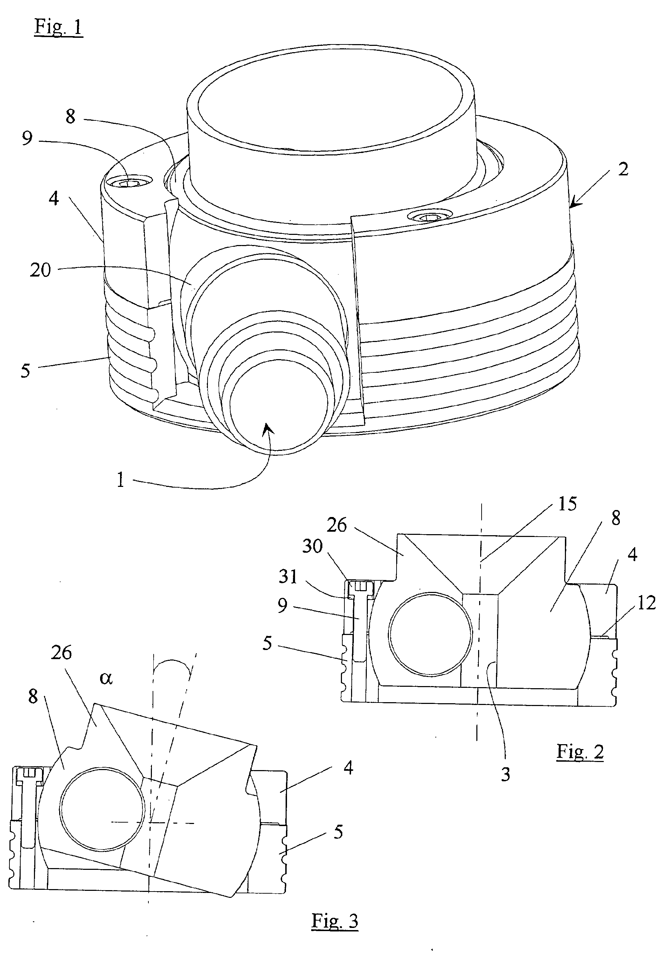

Figur 1 zeigt in einer isometrischen Darstellung ein als Gelenk ausgebildetes Verriegelungselement nach der Erfindung in einer isometrischen Darstellung. In der gezeigten Darstellung kann das Verriegelungselement z. B. für die Anbindung einer Prothese an einen Unter- und/oder Oberschenkelstumpf Verwendung finden.

Das Verriegelungselement nach Fig. 1 ist prothesenfest angeordnet und dient der Verriegelung mit einem über den Amputationsstumpf zu ziehenden Strumpf, einem Silikon-Liner, über einen strumpffesten Verriegelungsstift, regelmäßig als Verriegelungs-Pin bezeichnet, der in das Verriegelungselement eingeführt und dort von einer Einzugsmechanik 1 in einer Aufnahme 3 weiter eingezogen und verriegelt wird. Einzug und Verriegelung durch die Einzugsmechanik 1 an sich sind Stand der Technik.

Das Ausführungsbeispiel des Verriegelungselementes nach der Erfindung ist ein Gelenk, das eine Relativbewegung zwischen der Einzugsmechanik 1 bzw. dem von dieser in der Aufnahme 3 festgelegten Verriegelungsstift und einer prothesenfesten Aufnahme 2 in drei Freiheitsgraden zuläßt. Hierzu ist das Verriegelungselement mit einer Verbindung zwischen der Aufnahme 2 und der Einzugsmechanik 1 nach Art eines Kugelgelenkes versehen, nämlich mit einem Kugelschichtgelenk.

Dieses erlaubt beschränkte rotationssymmetrische Relativbewegungen, hier Kreisbewegungen, in allen Freiheitsgraden.

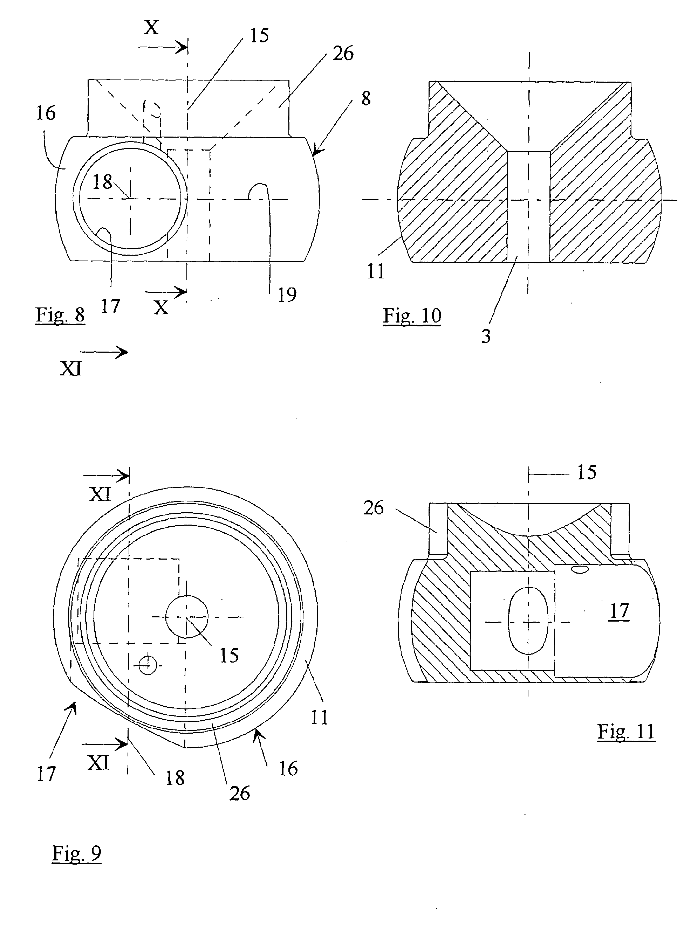

In konstruktiver Ausgestaltung ist das Verriegelungselement nach der Erfindung mit einer Aufnahme 2 versehen, die in ein Oberteil 4 und ein Unterteil 5 aufgeteilt ist. Das Oberteil 4 wie das Unterteil 5 weisen jeweils halbschalenartig ausgebildete Gleitflächen 6,7 auf, mit welchen die Aufnahme 2 einen Gelenkkörper 8 gemäß den Fig. 8 bis 11 einfaßt. Dieser Gelenkkörper 8 ist rotationssymmetrisch, hier kugelartig für das Einfassen durch die Gleitflächen 6,7 ausgebildet. Eine elliptische Ausbildung wäre gleichfalls möglich. Das Oberteil 4 und das Unterteil 5 sind insbesondere hinsichtlich der Gleitflächen 6,7 hier weitestgehend symmetrisch ausgeführt.

Der Gelenkkörper 8 ist weiter als Kugelschichtkörper 16 ausgeführt, d.h. zwei gegenüberliegende Polkappen fehlen.

Das Oberteil 4 und das Unterteil 5 sind durch Schrauben 9 miteinander verbunden. Die Köpfe 30 der Schrauben 9 sind in Ausnehmungen 10 versenkt im Oberteil 4 aufgenommen und werden jeweils gegen die Kraft einer Feder 31, bspw. als Druckfeder oder nach Art eines Sprengringes ausgebildet, festgezogen. Durch das Anziehen der Schrauben 9 kann die Reibungskraft zwischen den Gleitflächen 6,7 und der Kugelschichtmantelfläche 11 des Gelenkkörpers 8 eingestellt werden und wird durch das Maß dieser Reibungskraft bestimmt die möglichen Relativbewegungen zwischen dem Gelenkkörper 8 und der Aufnahme 2 abgebremst.

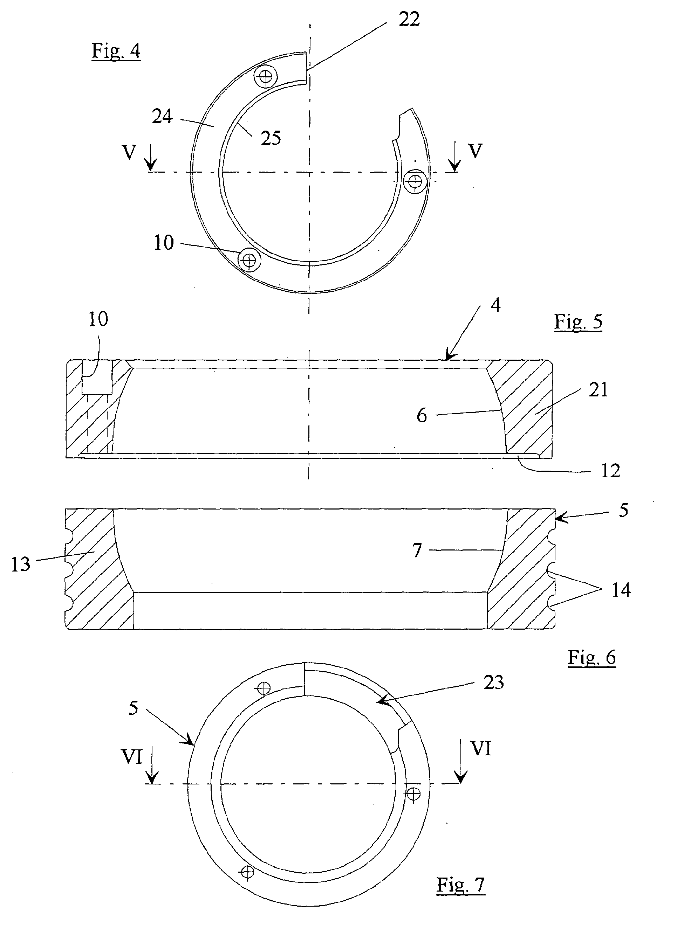

Damit durch das Anziehen der Schrauben 9 die Reibungskraft zwischen den Gleitflächen 6,7 und dem Gelenkkörper 8 in einem weiten Bereich einstellbar ist, weist das Oberteil 4 beim Ausführungsbeispiel eine die Gleitflächen 6,7 trennende, umlaufende Nutung 12 auf. Es ist die Nutung 12 radial so tief gehend, daß auch die Schrauben 9 die Nutung 12 frei durchsetzen, vgl. Fig. 2 und 3. Durch diese Maßnahme kann geradezu eine Klemmwirkung des Oberteils 4 auf den Gelenkkörper 8 erreicht werden.

Da die Aufnahme 2, wie auch der Gelenkkörper 8, bevorzugt aus einem Kunststoff, insbesondere Polyamid, besteht, kann es zweckmäßig sein, für die Schrauben 9 gesonderte Gewindeeinsätze in dem Unterteil 5 vorzusehen. Dies auch im Hinblick auf die Möglichkeit der exakten Einstellung der Kraft auf den Gelenkkörper 8 über die Gleitflächen 6,7.

Die umlaufende Mantelwand 13 des Unterteiles 5 weist eine Oberflächenstrukturierung für ein festes Einbringen in eine entsprechende Ausnehmung der Prothese auf, z.B. wie hier umlaufende Nuten 14, die eine hinterschneidende, formschlüssige Verbindung mit der Prothese in axialer Richtung, mit Bezug auf eine Symmetrieachse 15, ermöglichen.

Ein Auszug des Unterteiles aus der Prothese ist damit weitgehend ausgeschlossen und eine sichere Befestigung gegeben.

Der Gelenkkörper 8, vgl. Fig. 8 bis 11, weist einen zu einer Symmetrieachse 15 rotationssymmetrischen Kugelschichtkörper 16 auf. Dessen Kugelschichtmantelfläche 11 wird von den Gleitflächen 6,7 der Aufnahme 2 vollständig eingefaßt. Ein typischer Durchmesser für den Kugelschichtkörper 16 beträgt etwa 50 mm. Der Kugelschichtkörper 16 nimmt die Einzugs- und Ver- bez. Entriegelungsvorrichtung der Einzugsmechanik 1 in einer Aufnahmebohrung 17 auf, vgl. Fig. 1. Die Achse 18 dieser Aufnahmebohrung sowie die Symmetrieachse 15 sind Normale, d. h. senkrecht auf einer Ebene stehende Achsen, welche Ebenen bevorzugt aufeinander senkrecht stehen. Hier sind dies die Zeichenebene mit der Achse 18 als Normale und in der Darstellung gemäß Fig. 8 eine horizontale Ebene 19 als Normale der Achse 15.

In die Aufnahmebohrung 17 wird ein Abstandsring 20 für die Einzugs- und Ver- bez. Entriegelungsvorrichtung der Einzugsmechanik 1 in an sich herkömmlicher Technik eingebracht. Um einen Einbau derartiger, insbesondere stiftartig ausgebildeter Vorrichtungen zu ermöglichen, weisen die Mantelwände 21, 13 des Ober- 4 und des Unterteiles 5 hier jeweils eine Durchbrechung 22,23 jeweils auf. Das Umfangsmaß dieser Durchbrechung, vgl. Fig. 4 und 7, bestimmt die mögliche Rotation des Gelenkkörpers 8 um seine Symmetrie- und Drehachse 15 durch einen Anschlag des Abstandsrings 20 und/oder einer stiftartig ausgebildeten Einzugs- und Ver- bzw. Entriegelungsvorrichtung an den insbesondere senkrecht aufgehenden Rändern der Durchbrechungen 22,23. Durch diese Maßnahme wird die Relativbewegung um diese Achse 15 begrenzt.

Alternativ kann von einer herkömmlichen Einzugsmechanik, die zumeist ein verdrehbares Schneckenrad für ein Einziehen des dann eine entsprechende Zahnung aufweisenden Verriegelungs-Pin auch abgegangen werden, wenn bspw. ein gezahnter oder sogar glatter Verriegelungsstift vorgesehen wird, der von einer geeignet ausgebildeten, gelenkkörperfesten Klemmvorrichtung gehalten wird. Solche Klemmvorrichtungen mit Klemmkeilen, Klemmbügeln oder dergleichen, wie sie bspw. bei bekannten Kartuschenpistolen Verwendung finden, sind im Stand der Technik wohl bekannt. Eine Entriegelung einer solchen Klemmvorrichtung kann, wie beim Ausführungsbeispiel, tangential durch die Durchbrechungen 22,23 und die Aufnahmebohrung 17 vorgesehen werden, die dann erheblich kleiner ausfallen können, oder auch ober- und/oder unterhalb des Verriegelungselementes, wenn geeignete Umlenkvorrichtungen für einen Entriegelungsstift und/oder einen Entriegelungszug vorgesehen werden. Als zweckmäßig bei einer solchen Verriegelung hat es sich erwiesen, wenn für einen Einzug des Verriegelunsstiftes an diesem ein Hilfsmittel in Form einer Zugvorrichtung vorgesehen ist, die in die Aufnahme 3 für den Verriegelungsstift einbringbar und aus dieser wieder prothesenfest herausgeführt ist derart, daß durch Zug an einem freien Ende der Zugvorrichtung ein exakter Einzug des Verriegelungsstiftes in die Aufnahme 3 sichergestellt ist. Eine äußerst geringe Bauhöhe des Verriegelungselementes nach der Erfindung kann so erreicht werden, da ein im Durchmesser vergleichsweise großes Schneckenrad für den Einzug des Verriegelungsstiftes entfällt.

Weiter weist auch das Oberteil 4 in seiner Oberseite 24 eine Durchbrechung 25 auf, die von einem Ansatz 26 des Gelenkkörpers 8 durchsetzt wird. Das axiale Maß dieses Ansatzes 26 ist so gewählt, daß durch einen Anschlag des Ansatzes 26 an den Rand der Durchbrechung 25 die Relativbewegung auch in den zwei übrigen Freiheitsgraden begrenzt wird, vgl. Fig. 3. Es ist diese Begrenzung eine symmetrische, da die Durchbrechung 25 wie auch der Ansatz 26 kreissymmetrisch ausgebildet sind. Der maximal mögliche Öffnungswinkel 2α ist auf insgesamt etwa 30° begrenzt, womit aufgrund der Symmetrie gegenüber der Achse 15 eine Auslenkung α von etwa 15° möglich ist.

Der Ansatz 26 kann nach herkömmlicher Art der unmittelbaren Aufnahme eines Verriegelungsstiftes eines Stumpfes dienen und hierfür eine entsprechende Aufnahme 3 aufweisen oder ist eine solche Aufnahme an dem Ansatz 26 auch anschließbar.

## Patentansprüche

1. Prothesenfestes Verriegelungselement für eine Verriegelung zwischen einem über einen Amputationsstumpf zu ziehenden Strumpf und einer Prothese, mit einer Einzugsmechanik für einen an dem Strumpf fest angeschlossenen Verriegelungsstift, gekennzeichnet durch eine eine Relativbewegung zwischen der Einzugsmechanik (1) und einer prothesenfeste Aufnahme (2) zulassenden Verbindung derselben.

2. Verriegelungselement nach Anspruch 1, gekennzeichnet durch eine Relativbewegung mit drei Freiheitsgraden.

3. Verriegelungselement nach einem oder mehreren der vorangehenden Ansprüche, **dadurch gekennzeichnet**, daß die Relativbewegung eine rotationssymmetrische Komponente aufweist.

4. Verriegelungselement nach einem oder mehreren der vorangehenden Ansprüche, **dadurch gekennzeichnet**, daß die Relativbewegung eine kreissymmetrische Komponente aufweist.

5. Verriegelungselement nach einem oder mehreren der vorangehenden Ansprüche, **dadurch gekennzeichnet**, daß die Relativbewegung gebremst erfolgt.

6. Verriegelungselement nach einem oder mehreren der vorangehenden Ansprüche, **dadurch gekennzeichnet**, daß die Relativbewegung durch Reibungskraft gebremst wird.

7. Verriegelungselement nach einem oder mehreren der vorangehenden Ansprüche, **dadurch gekennzeichnet**, daß die Wege der Relativbewegung begrenzt sind.

8. Verriegelungselement nach einem oder mehreren der vorangehenden Ansprüche, **dadurch gekennzeichnet**, daß die Verbindung ein Gelenk ist.

9. Verriegelungselement nach einem oder mehreren der vorangehenden Ansprüche, **dadurch gekennzeichnet**, daß die Verbindung ein kardanisches Gelenk ist.

10. Verriegelungselement nach einem oder mehreren der vorangehenden Ansprüche, **dadurch gekennzeichnet**, daß die Verbindung ein Kugelgelenk ist.

11. Verriegelungselement nach einem oder mehreren der vorangehenden Ansprüche, **dadurch gekennzeichnet**, daß die Verbindung ein Kugelschichtgelenk ist.

12. Verriegelungselement nach einem oder mehreren der vorangehenden Ansprüche, **dadurch gekennzeichnet**, daß die Aufnahme (2) ein Ober- (4) und Unterteil (5) mit jeweils halbschalenartig ausgebildeten Gleitflächen (6, 7) aufweist zur Aufnahme eines rotationssymmetrischen, insbesondere kugelartigen, die Einzugsmechanik (1) aufweisenden Gelenkkörpers (8).

13. Verriegelungselement nach einem oder mehreren der vorangehenden Ansprüche, **dadurch gekennzeichnet**, daß Ober- (4) und Unterteil (5) miteinander verschraubt sind derart, daß über die Schrauben (9) die Reibungskraft zwischen den Gleitflächen (6, 7) und dem Gelenkkörper (8) einstellbar ist.

14. Verriegelungselement nach einem oder mehreren der vorangehenden Ansprüche, **dadurch gekennzeichnet**, daß die Schrauben (9) jeweils gegen die Kraft einer Feder (31) einschraubbar sind.

15. Verriegelungselement nach einem oder mehreren der vorangehenden Ansprüche, **dadurch gekennzeichnet**, daß zwischen dem Ober- (4) und dem Unterteil (5) wenigstens eine umlaufende Nutung (12) die Gleitflächen (6, 7) trennt.

16. Verriegelungselement nach einem oder mehreren der vorangehenden Ansprüche, **dadurch gekennzeichnet**, daß die Symmetrieachse (15) des Gelenkkörpers (8) und die Achse (18)einer stiftartig ausgebildeten Einzugs- und Ver- bez. Entriegelungsvorrichtung der Einzugsmechanik (1) Normale senkrecht aufeinander stehenden Ebenen sind.

17. Verriegelungselement nach einem oder mehreren der vorangehenden Ansprüche, **dadurch gekennzeichnet**, daß das Ober- (4) und/oder das Unterteil (5) eine Durchbrechung (22, 23) in ihrer Mantelwand (13,21) aufweisen für die Durchführung der Einzugs- bzw. Ver- und Entriegelungsvorrichtung der Einzugsmechanik (1).

18. Verriegelungselement nach einem oder mehreren der vorangehenden Ansprüche, **dadurch gekennzeichnet**, daß durch das Umfangsmaß der Durchbrechung(en) (22, 23) eine Rotation des Gelenkkörpers (8) um seine Symmetrieachse (15) begrenzt wird.

19. Verriegelungselement nach einem oder mehreren der vorangehenden Ansprüche, **dadurch gekennzeichnet**, daß das Oberteil (4) eine Durchbrechung (25) aufweist, die von einem Ansatz (26) des Gelenkkörpers (8) durchsetzt wird.

20. Verriegelungselement nach einem oder mehreren der vorangehenden Ansprüche, **dadurch gekennzeichnet**, daß der Ansatz (26) und die Durchbrechung (25) des Oberteils (4) rotationssymmetrisch, insbesondere kreissymmetrisch ausgebildet sind.

21. Verriegelungselement nach einem oder mehreren der vorangehenden Ansprüche, **dadurch gekennzeichnet**, daß die Aufnahme (2) und/oder der Gelenkkörper (8) aus einem Kunststoff, insbesondere aus einem Polyamid, bestehen.

22. Verriegelungselement nach einem oder mehreren der vorangehenden Ansprüche, **dadurch gekennzeichnet**, daß die Einzugsmechanik eine einen in eine Aufnahme (3) eingebrachten Verriegelungsstift gegen ein Ausziehen klemmend haltende Verriegelungsvorrichtung aufweist.

23. Verriegelungselement nach einem oder mehreren der vorangehenden Ansprüche, **dadurch gekennzeichnet**, daß die Verriegelungsvorrichtung einen Klemmbacken, einen Klemmbügel oder dergleichen aufweist, der durch eine Keilwirkung den Verriegelungsstift in seiner Aufnahme (3) selbsttätig klemmend festlegt.

24. Verriegelungselement nach einem oder mehreren der vorangehenden Ansprüche gekennzeichnet durch eine den Klemmbacken, den Klemmbügel oder dergleichen lösende Entriegelungsvorrichtung.

25. Verriegelungselement nach einem oder mehreren der vorangehenden Ansprüche, gekennzeichnet durch einen prothesenfesten Auslaß für ein an dem Verriegelungsstift angebundene Zugvorrichtung.
